Europäisches Patentamt

European Patent Office

Office européen des brevets

(11) Publication number: **0 364 637**
**A1**

# EUROPEAN PATENT APPLICATION

(21) Application number: 88309835.2

(51) Int. Cl.5: **A45D 24/10**

(22) Date of filing: **20.10.88**

(43) Date of publication of application:
**25.04.90 Bulletin 90/17**

(84) Designated Contracting States:
**AT BE CH DE ES FR GB GR IT LI LU NL SE**

(71) Applicant: **Wang, Lianji**
**Dalian Marine College**
**Dalian(CN)**

(72) Inventor: **Wang, Lianji**
**Dalian Marine College**
**Dalian(CN)**

(74) Representative: **Kerr, Simonne June et al**
**European Patent Attorney POTTS, KERR &**
**CO. P.O. Box 688**
**Ascot Berkshire SL5 8YT(GB)**

(54) **Magnetic comb.**

(57) A magnetic comb type dandruff removal hair
fixing device, which comprises permanent magnet, a
plurality of teeth in the form of inductive needles
adapted to contact the scalp is characterized in that
average surface strength of the permanent magnet
(2) is 1000 -1500 GS, the inductive needles (5) being
adapted to produce an inductive magnetic strength
of 250 - 1000 GS on average at their points, the
inductive needles being fixed on a base (4) positioned over the body of the permanent magnet (2).

EP 0 364 637 A1

This invention relates to a magnetic hair care device, or more specifically, a type of magnetic care comb.

Magnetic combs are known which comprise micromagnets within its teeth. Such a comb is effective to reduce dandruff and hair removal etc., after the hair has been combed. However, since the magnetic material of such a magnetic comb is positioned inside the teeth of the comb there is an obvious defect in its structure, namely difficulty in manufacture. It is very difficult to make the magnetic force of comb's teeth stronger, therefore one is left only with the choice of adopting expensive and rare materials and more advanced equipment in order to make it.

The comb made in accordance with the invention has a magnetic force field strength for the various skin diseases to be treated being mostly found at superficial portions of the scalp. It not only overcomes the defects of existing magnetic combs which are difficult to manufacture but also upgrades the curative effect.

An object of the invention is to provide improved permanent magnetic comb for removal of dandruff from the hair/scalp which comprises a simple structure, easy to manufacture and cheap to market.

Another object of the invention is to provide a type of magnetic cure tool which is effective to cure Seborrheic dermatitis, Pityriasis, migraine, hypertension and neuraesthesia etc., and also for stopping itchiness.

According to the present invention a magnetic comb type dandruff removal hair fixing device, comprising a permanent magnet a plurality of teeth in the form of inductive needles adapted to contact the scalp characterized in that average surface strength of the permanent magnet is 1000 - 1500 GS, the inductive needles being adapted to produce an inductive magnetic strength of 250 - 1000 GS on average at their points, inductive needles being fixed on a base positioned over the body of the permanent magnet.

For easy carrying by the user, the magnet body together with its attached base and fixed inductive needles are positioned into a casing with a cover. The height of the cover is adapted to the length of inductive needles.

For meeting the strength requirement of the magnetic field by different patients such as dandruff removal from the hair, fixing device can be made with different dosage strengths. The low dosage inductive needles may have an average magnetic strength of 250 - 400 GS at their points, and high dosage inductive needles may have an average magnetic strength of 400 - 1000 GS at their points. The length of inductive needles may be 15- 25 mm. The thickness of the body of the permanent magnet may be 14 - 22 mm and its diameter may be 35 - 45 mm.

For ease of manufacture, a permanent magnet body with a bigger surface strength may be chosen for making different dandruff removal dosages and a thick non-magnetic body and attached base of fixed inductive neeles to reach the strength of magnetic field requested by the points of the inductive needles.

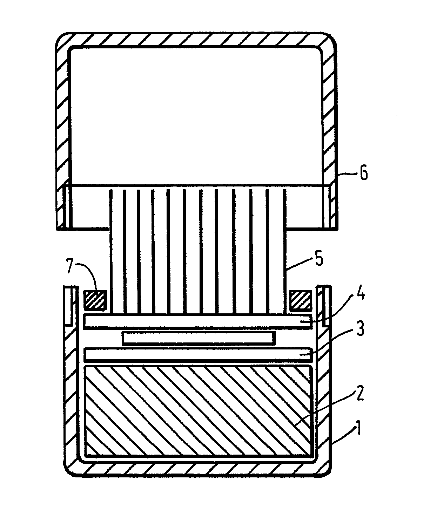

The invention will be described further, by way of example with reference to the accompanying drawings in which the single figure is a view of the comb in section.

The comb is a substantially cylindrical structure, comprising a casing 1 for a permanent magnet 2, the casing 1 being preferably of plastics material. The average surface magnetic strength of the permanent magnet 2 is about 1100 GS having a thickness of 10 mm and diameter of 40 mm. A non-magnetic sheet 3 of 2 mm thickness is arranged on top of the permanent magnet, which limits the average magnetic strength on the points of inductive needles 5 to about 300 GS. A base 4, the non-magnetic sheet 3 and the permanent magnet 2 are passed through a clip ring 7 to fix them inside the casing 1. The base 4 which carries the teeth in the form of inductive needles 5 can be made of flexible plastics material or rubber. The inductive needles are about 18 mm long. The casing 1 has a cover 6 to protect the needles 5.

## Claims

1. A magnetic comb type dandruff removal hair fixing device, which comprises permanent magnet, a plurality of teeth in the form of inductive needles adapted to contact the scalp, characterized in that average surface strength of the permanent magnet (2) is 1000 - 1500 GS, the inductive needles (5) being adapted to produce an inductive magnetic strength of 250 - 1000 GS on average at their points, the inductive needles being fixed on a base (4) positioned over the body of the permanent magnet (2).

2. A magnetic device according to claim 1, characterized in that the body of the permanent magnet (2) is 14 - 22 mm thick and its diameter is 35 - 45 mm, the inductive needles (5) being 15 - 25 mm long.

3. A magnetic device according to claim 1 or 2, characterized in that the body of the permanent magnet (2) together with the attached base (4) of inductive needles (5) are arranged inside a casing (1) having cover (6), the height of cover (6) being adapted to the length of inductive needles which it covers.

4. A magnetic device according to claim 3, characterized in that a non-magnetic sheet (3) is provided for adjusting the strength of the magnetic field on the points of the inductive needles (5) being placed between the body of the permanent magnet (2) and the attached base (4) of the fixed inductive needles (5).

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | FR-A-2607685 (COUDERC)<br>* the whole document *<br>--- | 1, 2 | A45D24/04 |
| Y | US-A-4480596 (SHUMIYASHU)<br>* column 4, line 65 - column 5, line 17; figures 6A, 6B *<br>--- | 1, 2 | |
| A | GB-A-429079 (CHAPMAN)<br>--- | | |
| A | DE-A-2022360 (TEZNER)<br>--- | | |
| A | FR-A-2551663 (TAMURA)<br>----- | | |
| | | | **TECHNICAL FIELDS SEARCHED (Int. Cl.5)**<br><br>A45D<br>A61N<br>A46B |

The present search report has been drawn up for all claims

| Place of search | Date of completion of the search | Examiner |
|---|---|---|
| THE HAGUE | 27 JUNE 1989 | SIGWALT C. |